Europäisches Patentamt

(19) European Patent Office

Office européen des brevets

(11) Publication number : **0 474 438 A1**

(12) **EUROPEAN PATENT APPLICATION**

(21) Application number : **91307943.0**

(22) Date of filing : **30.08.91**

(51) Int. CI.⁵ : **A61K 31/675, A61K 37/64**

(30) Priority : **04.09.90 US 577243**

(43) Date of publication of application :
**11.03.92 Bulletin 92/11**

(84) Designated Contracting States :
**AT BE CH DE DK ES FR GB GR IT LI LU NL SE**

(71) Applicant : **E.R. SQUIBB & SONS, INC.**
**Lawrenceville-Princeton Road**
**Princeton New Jersey 08543-4000 (US)**

(72) Inventor : **Sudilovsky, Abraham**
**712 Winchester Avenue**
**Lawrenceville, New Jersey (US)**

(74) Representative : **Thomas, Roger Tamlyn et al**
**D. Young & Co. 10 Staple Inn**
**London WC1V 7RD (GB)**

(54) **Method for preventing or treating cerebrovascular disease employing the ACE-inhibitor ceronapril.**

(57) A method is provided for preventing or treating cerebrovascular disease wherein the angiotensin converting enzyme inhibitor ceranapril is administered in an amount below the threshold level for lowering blood pressure, to prevent onset of cerebrovascular disease or during and/or after a cerebrovascular episode.

EP 0 474 438 A1

Jouve, 18, rue Saint-Denis, 75001 PARIS

This invention concerns the further medical uses of ceronapril, particularly those set out in the claims.

In accordance with the present invention, a method is provided for preventing or treating cerebrovascular disease, including preventing onset of cerebrovascular disease, and/or intervening during and/or after a cerebrovascular episode, in a mammalian species, wherein a therapeutically effective amount of the angiotensin converting enzyme (ACE) inhibitor ceronapril, which amount is below the threshold level for lowering systemic blood pressure, is systemically such as orally or parenterally, administered prior to, during or after a cerebrovascular episode, as soon after the episode as possible (starting at the acute phase), over a prolonged period.

The term "cerebrovascular disease" or "cerebrovascular episode" as employed herein refers to a transient ischemic attack or stroke including ischemic or hemorrhagic episodes, which involve an intra- or extracranial disturbance or interruption in arterial blood flow (cerebral infarction due to thrombosis or embolism from an atherosclerotic plaques or other cause).

The term "acute phase" of a stroke refers to the period immediately following a stroke during which there is a rapid onset of symptoms.

Ceronapril, which is employed in the method of the invention, is an angiotensin converting enzyme inhibitor having the chemical name (S)-1-[6-amino-2-[[hydroxy-(4-phenylbutyl)phosphinyl]oxy]-1-oxohexyl]-L-proline (also identified as SQ29,852) and is covered by U.S. Patent Nos. 4,452,790 and 4,745,196.

Where the patient to be treated in accordance with the present invention is normotensive or hypertensive, ceronapril will be administered in amounts below that required to cause hemodynamic effects, that is below that required to cause a significant reduction in blood pressure. By the term "significant" reduction (or lowering) in blood pressure is meant a reduction in blood pressure of at least about 10%.

Inasmuch as the amount of ceronapril employed will be below the threshold level for significantly lowering systemic blood pressure, it is clear that the mode of action of the ceronapril in the method of the invention is other than a cardiovascular effect and does depend on a change of systemic blood pressure. For example, it is theorized that the mode of action of the ceronapril may be augmentation of cerebral blood flow at the site of the lesion, protection at the cellular level and/or modulation of the ischemic injury cascade.

In carrying out the method of the present invention, the ceronapril may be administered to mammalian species, such as monkeys, dogs, cats, humans, etc., and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing ceronapril in tablets or capsules in an amount within the range of from about 0.5 to about 30 mg per day and preferably from about 10 to about 25 mg per day, which is below the usual dosage of a minimum of about 40 mg per day required to significantly lower blood pressure in a hypertensive patient.

For parenteral administration, ceronapril will be employed in an amount within the range of from about 0.3 mg to about 20 mg per day and preferably from about 6 mg to about 15 mg per day.

Regardless of mode of delivery or dosage form, the amount of ceronapril administered will be below that required to significantly lower systemic (diastolic) blood pressure in a hypertensive patient, that is a blood pressure lowering of less than about 10%.

The compositions described above may be administered in the dosage forms as described above in single or divided doses of one to four times daily. It may be advisable to start a patient on a low dose and work up gradually to a high dose.

Tablets of various sizes can be prepared, e.g., of about 2 to 500 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending ceronapril in a conventional liquid vehicle acceptable for pharmaceutical administration so as to provide the desired dosage in one to four teaspoonful.

Such dosage forms can be administered to the patient on a regimen of one to four doses per day.

In formulating the compositions, ceronapril, in an amount described above, is compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Some of the active substances described: above form commonly known, pharmaceutically acceptable salts such as alkali metal and other common basic salts or acid addition salts, etc. References to the base subatances are therefore intended to include those common salts known to be substantially equivalent to the parent compound.

The formumations as described above will be administered for a prolonged period, that is, for as long as the potential for cerebrovascular disease remains or the symptoms continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention.

Example 1

A ceronapril formulation suitable for oral administration in inhibiting onset of and/or treatings cerebrovascular disease during and/or after an attack is set out below.

1000 tablets each containing 10 mg of (S)-1-[6-amino-2-[[hydroxy(4-phenylbutyl)phosphinyl]oxy-1-oxohexyl]-L-proline were produced from the following ingredients.

| | |
|---|---|
| (S)-1-[6-Amino-2-[[hydroxy(4-phenyl-butyl)phosphinyl]oxy-1-oxohexyl]-L-proline (ceronapril) | 10 g |
| Corn starch | 50 g |
| Gelatin | 7.5 g |
| Avicel (microcrystalline cellulose) | 25 g |
| Magnesium stearate | 2.5 g |

The ceronapril and corn starch are admixed with an aqueous solution of the gelatin. The mixture is dried and ground to a fine powder. The Avicel and then the magnesium stearate are admixed with the granulation. This is then compressed in a tablet press to form 1000 tablets each containing 10 mg of active ingredient which is used for cerebrovascular disease as outlined above.

Example 2

1000 tablets each containing 20 mg of ceronapril are produced from the following ingredients:

| | |
|---|---|
| Ceronapril | 20 g |
| Lactose | 100 g |
| Avicel | 150 g |
| Corn starch | 50 g |
| Magnesium stearate | 5 g |

The ceronapril, lactose and Avicel are admixed, then blended with the corn starch. Magnesium stearate is added. The dry mixture is compressed in a tablet press to form 1000 505 mg tablets each containing 20 mg of active ingredient. The tablets are coated with a solution of Methocel E 15 (methyl cellulose) including as a color a lake containing yellow #6. The resulting tablets are useful in inhibiting onset of and/or treating cerebrovascular diasease during and/or after an episode.

Example 3

Two piece #1 gelatin capsules each containing 20 mg of ceronapril are filled with a mixture of the following ingredients:

| Ceronapril | 20 mg |
|---|---|
| Magnesium stearate | 7 mg |
| USP lactose | 193 mg. |

The resulting capsules are useful in inhibiting onset of cerebrovascular disease and/or treating cerebrovascular disease during and/or after an episode.

Example 4

An injectable solution for use in inhibiting onset of cerebrovascular disease and/or treating cerebrovascular disease during and/or after an episode is produced as follows:

| Ceronapril | 50 mg |
|---|---|
| Methyl paraben | 5 mg |
| Propyl paraben | 1 mg |
| Sodium chloride | 25 g |
| Water for injection qs. | 5 l. |

Ceronapril, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 10 mg of ceronapril per ml of solution for injection.

**Claims**

1.  Use of ceronapril for the manufacture of a medicament for preventing onset of or treating cerebrovascular disease.

2.  Use of ceronapril for the manufacture of a medicament for treatment of a cerebrovascular episode in a patient.

3.  Use of ceronapril for the manufacture of a medicament for treatment of a post-stroke condition in a patient.

4.  Use of ceronapril for the manufacture of a medicament for preventing onset of cerebrovascular disease.

5.  Use as defined in any preceding claim wherein the ceronapril is to be administered in an amount below the threshold level for significantly lowering blood pressure in a hypertensive patient.

6.  Use as defined in any preceding claim wherein the ceronapril is to be administered orally.

7.  Use as defined in claim 6 wherein the ceronapril is to be administered in an amount within the range of from about 0.5 to about 30 mg per day.

8.  Use as defined in claim 7 wherein the ceronapril is to be administered in an amount within the range of from about 10 to about 25 mg per day.

9.  Use as defined in any one of claims 1-5 wherein the ceronapril is to be administered parenterally in an amount within the range of from about 0.3 to about 20 mg per day.

10. Use as defined in claim 9 wherein the ceronapril is to be administered parenterally in an amount within the range of from about 6 to about 15 mg per day.

11. Use as defined in any preceding claim wherein ceronapril is to be administered to a normotensive patient.

European Patent Office

**PARTIAL EUROPEAN SEARCH REPORT**

which under Rule 45 of the European Patent Convention shall be considered, for the purposes of subsequent proceedings, as the European search report

Application Number

EP 91 30 7943

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | MEDICAL JOURNAL OF KINKI UNIVERSITY, vol. 15, no. 4 suppl., 1990, pages 47-50; H. SHIOKAWA et al.: "The effects of antihypertensive drugs and diet on M-SHRSP. Preventive and therapeutic effects when administered at different stages of life" * Abstract * --- | 1-4,6 | A 61 K 31/675 A 61 K 37/64 |
| X | KIDNEY INTERNATIONAL, vol. 37, no. 1, January 1990, page 394; C.T. STIER, Jr. et al.: "Dose-dependent protective effects of SQ-29852 against stroke and hypertensive renal disease in stroke-prone SHR" * Abstract * --- -/- | 1-6,11 | |

**TECHNICAL FIELDS SEARCHED (Int. Cl.5)**

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely : 7-10

Claims not searched :

Reason for the limitation of the search:

Method for treatment of the human or animal body by surgery or therapy (see article 52(4) of the European Patent Convention).

See sheet -C-

| Place of search | Date of completion of the search | Examiner |
|---|---|---|
| THE HAGUE | 04-11-1991 | KRAUTBAUER B. |

EPO FORM 1503 03.82 (P0407)

European Patent
Office

PARTIAL EUROPEAN SEARCH REPORT

Application Number

EP   91 30 7943

| | DOCUMENTS CONSIDERED TO BE RELEVANT | | CLASSIFICATION OF THE APPLICATION (Int. Cl. 5) |
|---|---|---|---|
| Category | Citation of document with indication, where appropriate, of relevant passages | Relevant to claim | |
| X | JOURNAL OF CARDIOVASCULAR PHARMACOLOGY, vol. 14, no. 5, 1989, pages 722-729, Raven Press, Ltd, New York, US; G. WALDEMAR et al.: "Angiotensin-converting enzyme inhibition and regional cerebral blood flow in acute stroke" * Summary; pages 722,723,725,727,728 * --- | 1-6,11 | |
| X | THERAPIE, vol. 43, no. 4, June/July 1988, pages 247-249, FR; C. CAPDEVILLE et al.: "Les inhibiteurs de l'enzyme de conversion assurent-ils une meilleurs prévention des accidents vasculaires cérébraux chez l'hypertendu?" * The whole article * ----- | 1-4,6, 11 | TECHNICAL FIELDS SEARCHED (Int. Cl.5) |

EPO FORM 1503 03.82 (P0410)

EP   91 30 7943   -C-

Claims searched incompletely  1-11 :


The subject matter of claims 1-11 are not supported by
pharmacological evidence. In the absence of pharmacological
data the evaluation of the technical nature of the subject
matter and of the prior art is equivocal and subjective. As
a consequence it may well be that relevant prior art has
not been retrieved.